# EUROPEAN PATENT APPLICATION

(11) **EP 1 859 740 A1**
(43) Date of publication of application: **28.11.2007**
(21) Application number: 06425364.4
(22) Date of filing: 26.05.2006
(51) Int. Cl.: A61B 6/14, G03B 42/04

(54) **System for positioning radiographic material for performing intraoral X-rays**

(71) Applicant: Madiai, Valter, 11100 Aosta (IT)
(72) Inventor: Madiai, Valter, 11100 Aosta (IT)
(74) Representative: Gerbino, Angelo

(57) **Abstract**

The system for positioning radiographic material (9) for performing intraoral X-rays comprises: a sleeve part (10) having a longitudinal axis (14) and a distal portion (20); an annular member (12) mounted in a coaxially rotatable manner around the distal portion (20) of the sleeve part (10) and having at least one seat (40a, 40b) for receiving an end arm (42) of a support (44) for the radiographic material (9); and means for selectively fixing the annular member (12) in a predefined rotational configuration with respect to the longitudinal axis (14) of the sleeve part (10).

## Description

The present invention relates to a system for positioning radiographic material for performing intraoral X-rays.

This material may be, for example, a radiographic film or an LCD sensor, in the case where digital methods are used, and must be positioned by means of a support adjacent to the part of the dental arch to be x-rayed so that it can be irradiated by means of an X-ray tube.

According to a known technique, the radiographic material is kept in a fixed position by means of the patient biting on a special part of the associated support, while the dentist manoeuvres separately the X-ray tube so as to direct the radiation towards the radiographic material. Ideally the latter should be struck at right angles by the incident radiation. In practice, however, it is difficult to ensure in a constant and absolute manner this right-angled condition, with the result that the quality of the X-ray obtained may be affected.

In any case, the need for the patient to have to bite on the radiographic material support and therefore keep his/her mouth closed represents a critical factor in this known technique. In fact, in this condition the radiographic material itself may cause the patient pain as a result of contact with the mucous membrane of the palate or the lingual floor. In endodontics, where endodontic instruments are positioned in the dental canals, it is not possible to perform the operation described above, whereas, when instruments such as a "rubber dam" are present, it is necessary to remove some parts beforehand in order to be able to proceed. In surgery where wound flaps are open, due to extraction, cysts, apicoectomy and even more so in the case of implantology, the requirement for the patient to keep his/her mouth closed may be a limiting factor or, in some cases, even not possible. Finally, in the case of edentulous patients or patients with handicaps, having to continuously bite on the radiographic material support is problematic.

The object of the present invention is to provide a positioning system which overcomes the abovementioned drawbacks of the prior art.

According to the invention, this object is achieved by means of a positioning system having the features mentioned specifically in the claims which follow.

During use, the sleeve of the system according to the invention is associated coaxially with an X-ray tube of an X-ray apparatus, while the radiographic material is kept by the support in a position at right angles to the continuation of the common longitudinal axis of the sleeve part and the X-ray tube, ensuring perfect alignment in an automatic and continuous manner.

The dentist may then insert the distal part of the support with the radiographic material inside the mouth kept open by the patient and position, as desired, this distal part by rotating the annular member with respect to the sleeve part and conveniently directing the X-ray tube, without this directional manoeuvre being subject to any substantial external constraints. The fact that the patient must keep his/her mouth open constitutes a major advantage since the necessary space is provided for the radiographic procedure even where other devices are present. Therefore, in endodontics, it is possible to perform X-rays without having to remove the endodontic instruments beforehand, whereas in surgery and implantology it is possible to perform X-rays while keeping the various diagnostic and operating equipment in position, with an obvious reduction in the time required and stress suffered by the patient.

Performing an open-mouth X-ray procedure according to the invention is moreover particularly simplified compared to conventional X-ray procedures - which require a biting action - in the case of edentulous patients or patients with handicaps.

Further advantages and characteristic features of the present invention will emerge from the detailed description which follows, provided by way of a non-limiting example with reference to the accompanying drawings in which:
Figure 1 is a perspective view of a positioning system according to the invention, inside a seat of which an end arm of a radiographic material support is inserted,
Figure 2 is a perspective view of the positioning system according to Figure 1 with an end arm of another radiographic material support inserted inside a different seat,
Figure 3 is a partially cross-sectioned side view of a sleeve element forming part of the positioning system according to the invention,
Figure 4 is a plan view, from the rear, of an annular member forming part of the positioning system according to the invention, and
Figure 5 shows schematically the positioning system according to the invention associated with an X-ray tube in the operating condition.

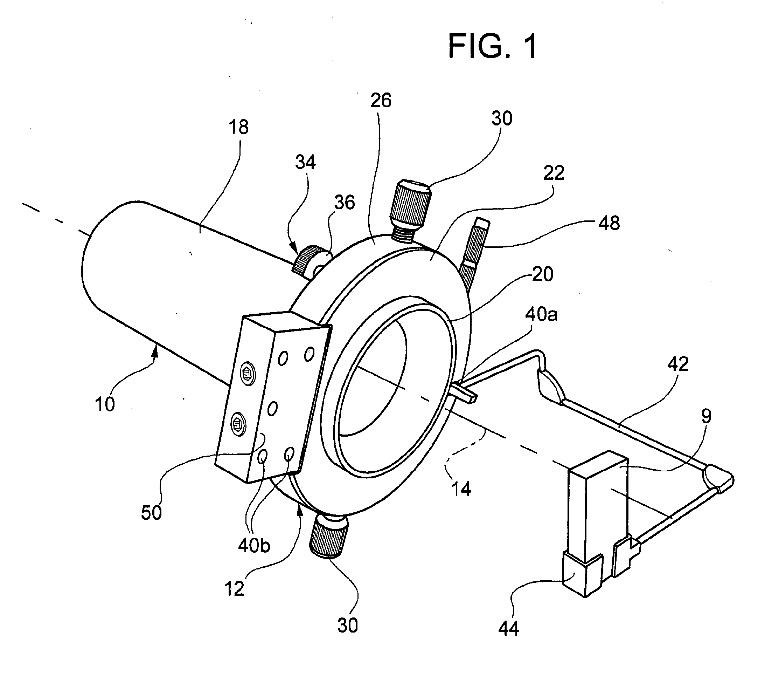

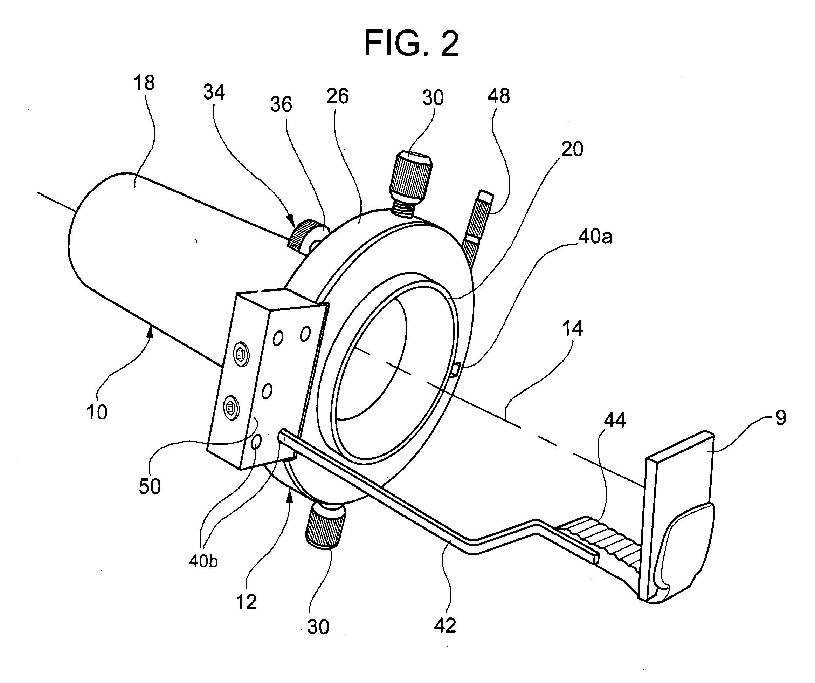

A system for positioning radiographic material 9 in order to perform intraoral X-rays comprises (Figs. 1 or 2) a sleeve part 10, an annular member 12 and means for selectively fixing the annular member 12 in a predefined rotational configuration with respect to the sleeve part 10.

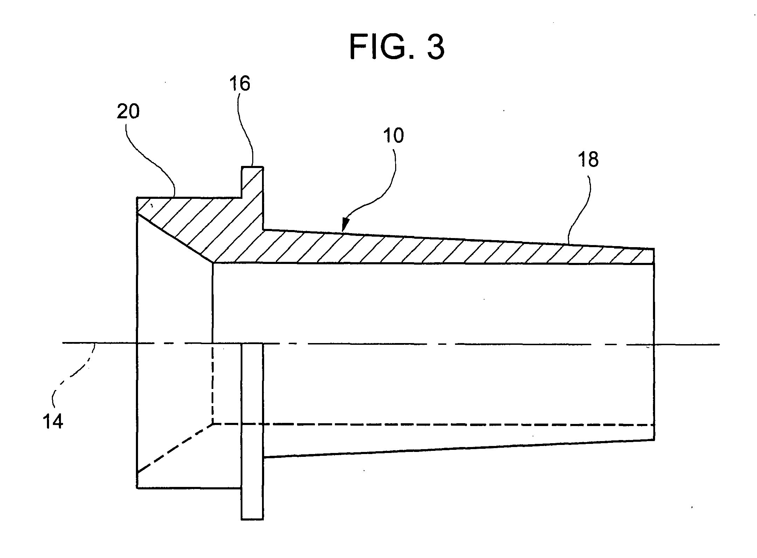

The sleeve part 10 (Fig. 3) has a longitudinal axis 14 and has a radially projecting flange 16 positioned between a proximal portion 18 and a distal portion 20. The latter has a cylindrical outer surface, while the proximal portion 18 has a frustoconical outer surface.

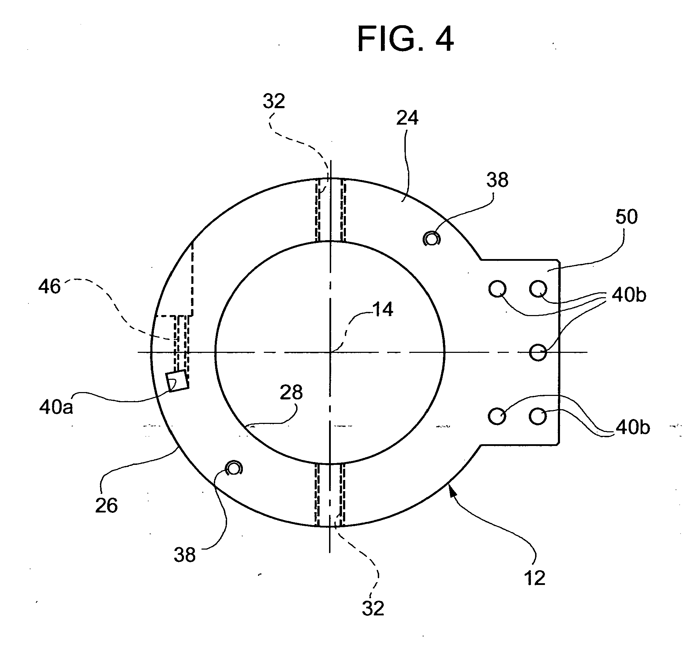

The annular member 12 (Fig. 4) is shaped substantially in the manner of a circular rim having a main front face 22, a main rear face 24, an outer side surface 26 and a circular inner side surface 28. It is mounted in a coaxially rotatable manner around the distal portion 20 of the sleeve part 10 and the axis 14, bearing against the flange 16.

The means for selectively fixing the annular member 12 relative to the sleeve part 10 in a predefined rotational configuration with respect to the axis 14 comprise two screws 30 which can be screwed inside respective threaded through-holes 32 extending radially between the outer and inner side surfaces 26, 28 of the annular member 12. Advantageously, the two holes 32 are arranged in diametrically opposite positions.

Means for selectively retaining the annular member 12 and the sleeve member 10 in the axial direction are also present. These selective retaining means comprise two screws 34 (only one of which can be seen in the figures) which have an eccentric head 36 and can be screwed inside a respective blind threaded hole 38 formed in the main rear face 24 of the annular member 12 and externally flush with the flange 16. Advantageously the two holes 38 are arranged in diametrically opposite positions.

The annular member 12 has, moreover, formed therein seats 40a, 40b extending in the axial direction for receiving (Figs. 1 and 2) end arms 42 of supports 44 - known per se and conventional - for the radiographic material 9. In particular, a seat 40a is formed in the manner of a cavity open towards the outside and is connected with the outer side surface 26 of the annular member 12 by a threaded hole 46 inside which the shank of a screw 48 which acts as a means for selectively retaining the end arm 42 is screwed. Other seats 40b are instead formed in a projection 50 of the annular member 12. The seats 40b are not provided with specific means for retention, but the latter is ensured by the friction existing between the walls of the seat 40b and the surface of the end arm 42 inserted therein.

During use of the positioning system according to the invention, the radiographic material 9 to be irradiated is mounted on the support 44, the end arm 42 thereof being inserted in one of the seats 40a or 40b (cf. Figure 1 and 2 respectively) of the annular member 12 and locked there by screwing the screw 48 inside the hole 46 or by means of friction.

By rotating the screws 34 so that the most radially projecting parts of the respective eccentric heads 36 engage with the rear face of the flange 16, extraction in the radial direction of the annular member 12 with respect to the sleeve part 10 is prevented. For their part, the screws 30 are not screwed fully inside the respective holes 32, such that the annular member 12 may freely rotate around the distal portion 20 of the sleeve part 10, remaining axially constrained thereto.

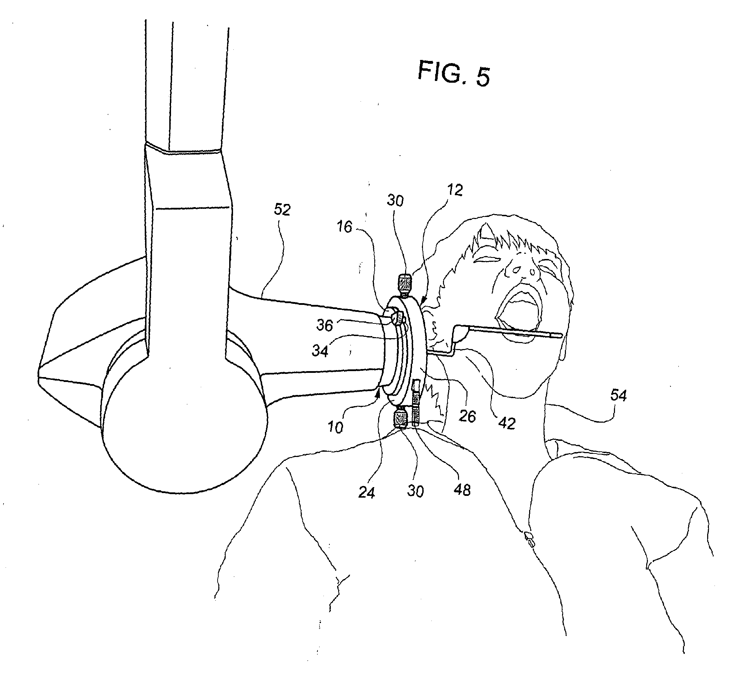

The proximal portion 18 of the sleeve part 10 is then inserted (Fig. 5) inside an X-ray tube 52 of an X-ray apparatus so that the radiation which is produced by the tube 52 is able to spread through the internal cavity of the part 10 parallel to the longitudinal axis 14 and strike at right angles against the radiographic material 9, kept by the support 44 along the continuation of the axis 14, so as to ensure perfect alignment in an automatic and continuous manner.

At this point, the operator is able to introduce the radiographic material supported by its support 44 inside the open mouth of the patient 54 and position it as desired, conveniently spatially directing the X-ray tube 52 and rotating the annular member 12 around the distal portion 20 of the sleeve part 10. The fact that, during this procedure, the patient 54 must keep his/her mouth open is of great help since ample manoeuvring space is ensured also where endodontic instruments or diagnostic and operating apparatus are already mounted in position (these would have in fact hindered, if not prevented entirely, the use of conventional positioning devices). Once the annular member 12 has been arranged around the sleeve part 10 in the desired optimum rotational configuration with respect to the longitudinal axis 14, this configuration is fixed by screwing the screws 30 inside the respective holes 32 so that the distal ends of the respective shanks bear against the outer surface of the distal portion 20 of the sleeve part 10.

In these conditions it is therefore possible to carry out the actual X-ray procedure which is performed in a manner conventional per se by means of irradiation of the radiographic material 9. Once this procedure has been completed, the support 44 may be extracted from the mouth of the patient 54 and separated from the annular member 12 in order to recover the radiographic material 9 which has been irradiated. Moreover, by loosening the screws 30 and 34, it is possible to remove the annular member 12 from the sleeve part 10, so as to be able to sterilize it subsequently with a view to carrying out another X-ray procedure on a new patient.

Obviously, without affecting the principle of invention, the constructional details and the embodiments may be widely varied with respect to that described purely by way of example, without thereby departing from the scope of the invention as defined in the accompanying claims. In particular, the sleeve part could be formed integrally with the X-ray tube, instead of as a separate part.

## Claims

1. System for positioning radiographic material (9) for performing intraoral X-rays, comprising:
- a sleeve part (10) having a longitudinal axis (14) and a distal portion (20),
- an annular member (12) mounted in a coaxially rotatable manner around the distal portion (20) of said sleeve part (10) and having at least one seat (40a, 40b) for receiving an end arm (42) of a support (44) for said radiographic material (9), and
- means for selectively fixing the annular member (12) in a predefined rotational configuration with respect to the longitudinal axis (14) of the sleeve part (10).

2. System according to Claim 1, in which said selective fixing means comprise at least one screw (30) which can be screwed inside a threaded through-hole (32) extending radially through said annular member (12).

3. System according to Claim 2, in which said selective fixing means comprise two screws (30) which can be screwed inside respective threaded through-holes (32) preferably arranged in diametrically opposite positions.

4. System according to any one of the preceding claims, in which said at least one seat (40a) is provided with means for selectively retaining said end arm (42).

5. System according to any one of the preceding claims, in which said annular member (12) is shaped substantially in the manner of a rim having a main front face (22), a main rear face (24), an outer side surface (26) and an inner side surface (28).

6. System according to Claims 4 and 5, in which said at least one seat (40a) is formed in the manner of a cavity open towards the outside and extending axially, said cavity being connected to the outer side surface (26) of the annular member by a threaded hole (46) inside which the shank of a screw (48) is screwed so as to allow the selective retention of said end arm (42) inside the seat (40a).

7. System according to any one of the preceding claims, in which said sleeve part (10) has a radially projecting flange (16) positioned between a proximal portion (18) and the distal portion (20), said flange (16) acting as a stop surface for said annular member (12).

8. System according to Claim 7, in which said distal portion (20) of the sleeve part (10) has a cylindrical outer surface, while said proximal portion (18) has a frustoconical outer surface.

9. System according to any one of the preceding claims, comprising means for selectively retaining in the axial direction said annular member (12) and said sleeve part (10).

10. System according to Claims 5, 7 and 9, in which said selective retaining means comprise at least one screw (34) which can be screwed inside a respective blind threaded hole (38) formed in the main rear face (24) of the annular member (12) and externally flush with said flange (16), said screw (34) having an eccentric head (36).

11. System according to Claim 10, in which said selective retaining means comprise two screws (34) which can be screwed inside respective blind threaded holes (38) preferably arranged in diametrically opposite positions.
